(19) Europäisches Patentamt
European Patent Office
Office européen des brevets

(11) **EP 1 967 160 B1**

(12) **FASCICULE DE BREVET EUROPEEN**

(45) Date de publication et mention
de la délivrance du brevet:
**25.02.2015 Bulletin 2015/09**

(51) Int Cl.:
*A61L 27/44* *(2006.01)*  *A61L 27/46* *(2006.01)*
*C03C 3/097* *(2006.01)*  *C03C 4/00* *(2006.01)*
*C03C 14/00* *(2006.01)*  *C08J 3/215* *(2006.01)*
*A61L 27/58* *(2006.01)*

(21) Numéro de dépôt: **08356024.3**

(22) Date de dépôt: **13.02.2008**

(54) **Procédé de préparation d'un matériau composite**

Verfahren zur Herstellung eines Verbundmaterials

Method of preparing a composite material

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **15.02.2007 FR 0701107**

(43) Date de publication de la demande:
**10.09.2008 Bulletin 2008/37**

(73) Titulaire: **Noraker**
**69100 Villeurbanne (FR)**

(72) Inventeurs:
• **Zenati, Rachid**
**69250 Neuville sur Saône (FR)**
• **Pacard, Elodie**
**69005 Lyon (FR)**

(74) Mandataire: **Brédeville, Odile Marie et al**
**Cabinet Germain & Maureau**
**BP 6153**
**69466 Lyon Cedex 06 (FR)**

(56) Documents cités:
WO-A-01/32072    WO-A-01/82989
US-A- 4 525 495    US-A- 5 977 204
US-A1- 2002 115 742

• ZHANG K ET AL: "Processing and properties of
porous poly(l-lactide)/bioactive glass
composites", BIOMATERIALS, ELSEVIER
SCIENCE PUBLISHERS BV., BARKING, GB, vol.
25, no. 13, 1 June 2004 (2004-06-01), pages
2489-2500, XP004486282, ISSN: 0142-9612, DOI:
10.1016/J.BIOMATERIALS.2003.09.033
• ITOH S ET AL: "Development of an artificial
vertebral body using a novel biomaterial,
hydroxyapatite/collagen composite",
BIOMATERIALS, ELSEVIER SCIENCE
PUBLISHERS BV., BARKING, GB, vol. 23, no. 19,
1 October 2002 (2002-10-01), pages 3919-3926,
XP004374384, ISSN: 0142-9612, DOI:
10.1016/S0142-9612(02)00126-6

**Description**

La présente invention porte sur un procédé de préparation d'un matériau composite de composition homogène comprenant une phase céramique bioactive et au moins un polymère biorésorbable.

Dans le domaine médical, en particulier dans le domaine des dispositifs médicaux implantables pour les applications de substitution osseuse, on cherche de plus en plus à obtenir des dispositifs médicaux implantables biorésorbables dans le corps humain ou animal avec des propriétés biologiques et mécaniques intéressantes.

Dans la présente demande, on entend par « biorésorbable » la caractéristique selon laquelle un matériau est absorbé par les tissus biologiques et disparaît in vivo au bout d'une période donnée, par exemple en moins de 24 mois, ou encore en moins de 8 semaines, ou encore en moins de quelques jours.

En effet, ces dispositifs médicaux implantables étant appelés, par exemple dans le cas de la subsitution osseuse, à être au contact d'os, il est souhaitable qu'ils présentent des propriétés biologiques telles que l'ostéoconduction ou l'ostéo-intégration, c'est-à-dire la capacité à promouvoir la croissance des cellules ostéoblastes.

Toutefois, compte tenu de leur fonction, par exemple de substitution ou de fixation, ces dispositifs médicaux implantables doivent également présenter une très bonne résistance mécanique. Par ailleurs, dans le cas où ces dispositifs médicaux implantables sont des moyens de fixation d'autres dispositifs médicaux implantables, comme par exemple des vis de fixation, ils ne doivent endommager ni les dispositifs qu'ils sont appelés à fixer ni les tissus humains avoisinants, et ce quelle que soit leur forme.

Dans ce but, on a cherché à réaliser des matériaux composites à base d'une part d'une phase organique biorésorbable et d'autre part d'une phase céramique.

Par phase céramique, on entend, au sens de la présente demande, une phase minérale choisie dans le groupe comprenant les céramiques, les vitrocéramiques, les verres et leur mélanges.

Dans un tel matériau composite, le but de la phase céramique est d'apporter la résistance mécanique et les propriétés biologiques nécessaires. Ainsi, il est nécessaire que cette phase céramique soit présente dans le matériau composite à une teneur minimale suffisante.

Ainsi, le document US 5, 977, 204 décrit un matériau composite comprenant une phase organique et une phase céramique, cette dernière pouvant représenter de 10 à 70% en volume, du volume du matériau.

Le document US 4, 192, 021 décrit un matériau composite solide comprenant une phase organique et une phase céramique, dans lequel le taux quantitatif de phase minérale par rapport à la phase organique est compris entre 10 :1 et 1 :1.

Le document WO 01/32072 décrit un implant biodégradable ayant un profil de dégradation biphasique, comprenant un polymère biodégradable dans lequel est dispersée une céramique biodégradable.

Le document US 2002/115742 décrit un matériau composite comprenant un polymère et une phase céramique bioactive sous forme de particules de taille moyenne inférieure ou égale à 500 nm.

Le document ZHANG K et AL : « Processing and properties of porous poly(l-lactide)/bioactive glass composites », BIOMATERIALS, vol.25, n°13, 1 juin 2004, p 2489-2500) décrit un procédé de préparation d'un composite poreux de polymère poly(l-lactide)(PLLA)/verre bioactif.

Le document ITOH S et AI : « Development of an artificial vertebral body using a novel biomaterial, hydroxyapatite/collagen composite », BIOMATERIALS, vol. 23, n° 19, 1 octobre 2002, pages 3919-3926 décrit un procédé de préparation d'un implant vertébral à base d'un composite hydroxyapatite/collagène.

Toutefois, ces matériaux de l'art antérieur se présentent généralement sous la forme de composés massifs solides peu homogènes. De tels matériaux ne donnent pas satisfaction lorsqu'ils sont utilisés dans la fabrication de dispositifs médicaux implantables, en particulier de dispositifs médicaux implantables de formes complexes, comme par exemple des vis de fixation. En particulier, de tels matériaux sont très difficilement transformables par les procédés de transformation connus, tels que le moulage par injection, injection transfert, compression ou extrusion. En effet, lorsqu'on cherche à transformer ces matériaux par un de ces procédés, il se produit généralement une ségrégation de la phase organique et de la phase céramique lors de l'opération de chauffage avant transformation. Ainsi, la phase céramique généralement se sépare de la phase organique empêchant ainsi un fonctionnement normal de la machine : il n'est alors pas possible d'obtenir le produit souhaité.

Ainsi, il subsiste le besoin d'un matériau composite comprenant une phase organique biorésorbable et une phase céramique, ledit matériau composite présentant une composition homogène pour permettre la fabrication de dispositifs médicaux implantables, en particulier de dispositifs médicaux implantables de formes complexes, par exemple par des procédés de transformation nécessitant une étape de préchauffage, ladite phase céramique étant présente dans ledit matériau composite en quantité suffisante pour garantir aux dispositifs médicaux implantables résultant les propriétés biologiques et mécaniques nécessaires à la fonction qu'ils sont appelés à exercer lors de leur utilisation, par exemple comme éléments de fixation et d'ancrage, dans le cas de vis d'interférence, de substituts osseux implantés.

La présente invention remédie à ce besoin en proposant un matériau composite et le procédé de fabrication d'un tel matériau, ce matériau étant apte à être transformé, en particulier par des procédés de transformation nécessitant une étape préalable de chauffage dudit matériau, pour produire des dispositifs médicaux implantables, en particulier des dispositifs médicaux implantables de formes complexes, de telle sorte que les dispositifs médicaux implantables ainsi produits présentent des propriétés biologiques et mécaniques particulièrement avantageuses pour leur utilisation dans le domaine médical, par exemple comme éléments de fixation résistants et résorbables pour des substituts osseux.

La présente invention porte sur un procédé de préparation d'un matériau composite de composition homogène tel que revendiqué dans la revendication 1.

Dans la présente demande, on entend, par « composition homogène » en ce qui concerne le matériau composite ou la dispersion visqueuse, le fait que les différents composants sont répartis de façon régulière au sein du volume constitué par ledit matériau composite ou ladite dispersion visqueuse. En particulier, dans le matériau composite selon l'invention, la phase céramique est de préférence sous la forme de particules et la phase organique, c'est-à-dire le polymère, est de préférence sous la forme d'une matrice, les particules de céramique étant dispersées de façon régulière au sein de la matrice organique.

Par matériau « bioactif », on entend, au sens de la présente demande, un matériau capable de développer une réponse biologique à l'interface entre ledit matériau et les tissus humains, et donc de développer une liaison entre ledit matériau et lesdits tissus humains.

Par « verre bioactif », on entend au sens de la présente invention un verre amorphe, partiellement ou totalement recristallisé, compatible avec le corps humain ou animal, et bioactif au sens tel que mentionné ci-dessus.

La présente demande décrit un matériau composite de composition homogène obtenu selon le procédé décrit ci-dessus. De préférence, le matériau composite comprend au moins 5% en poids, de préférence de 30% à 80%, en poids, d'une phase céramique bioactive, par rapport au poids total du matériau.

De préférence, ce matériau composite est sous la forme de granules.

Dans la présente demande, on entend, par « granule » une particule solide, poreuse ou non, de forme sensiblement sphérique. De préférence, les granules selon l'invention présentent un diamètre moyen allant de 0,1 à 5 mm, de préférence encore de 0,3 à 2 mm.

Le matériau composite en particulier lorsqu'il est sous la forme de granules, est particulièrement adapté à être transformé par des techniques de transformation ou de mise en forme nécessitant au moins une étape de chauffage dudit matériau, telles que le moulage par injection, injection transfert, compression ou extrusion.

La présente demande décrit l'utilisation d'un matériau composite tel que ci-dessus pour la, ou dans un procédé de, fabrication de dispositifs médicaux implantables par une technique de transformation nécessitant au moins une étape de chauffage dudit matériau.

La présente demande décrit un procédé de fabrication d'un dispositif médical implantable, comprenant les étapes suivantes :

- 1°) on dispose d'un matériau composite tel que décrit ci-dessus,
- 2°) on chauffe ledit matériau composite pour obtenir une pâte homogène,
- 3°) on coule ladite pâte homogène dans un moule,
- 4°) après refroidissement, le dispositif médical implantable est obtenu par démoulage.

La présente demande décrit des dispositifs médicaux implantables susceptibles d'être obtenus par un tel procédé. Ces dispositifs médicaux peuvent comprendre au moins 30%, de préférence entre 50 et 80%, en poids d'une phase céramique bioactive, par rapport au poids total du dispositif médical implantable.

Le matériau composite permet d'utiliser les techniques de moulage par injection, moulage par injection transfert, moulage par compression, par extrusion ou par usinage microtechnique pour la mise en forme de dispositifs médicaux implantables, de préférence biorésorbables, de toutes formes, même les plus complexes. Du fait de la composition particulièrement homogène du matériau composite les dispositifs médicaux implantables obtenus présentent des propriétés biologiques et mécaniques particulièrement avantageuses pour leur utilisation dans le domaine médical, en particulier dans le domaine de l'orthopédie, du rachis, du crano-maxillofacial, du dentaire et de la traumatologie.

Les dispositifs médicaux implantables obtenus à partir du matériau composite présentent en particulier une proportion en phase céramique particulièrement importante. De tels dispositifs médicaux implantables sont ainsi particulièrement résistants mécaniquement. Ainsi, il est possible de réaliser des dispositifs médicaux implantables, de préférence biorésorbables, de toutes formes, même complexes, et d'utiliser ces dispositifs médicaux implantables, de préférence biorésorbables, pour leurs propriétés mécaniques, par exemple comme éléments de fixation et d'ancrage. En particulier, il est possible de réaliser des dispositifs médicaux implantables résorbables tels que les vis d'interférence, les pines, les cages intervertébrales cervicales et lombaires, les plaques cervicales, les ancres, les agrafes.

Les dispositifs médicaux implantables obtenus à partir du matériau composite selon l'invention présentent également des propriétés biologiques remarquables : en particulier, du fait de leur haute teneur en phase céramique, ils sont capables de promouvoir l'ostéoconduction et/ou l'ostéointégration.

Selon une première étape du procédé de préparation du matériau composite selon l'invention, à savoir l'étape a°), on dispose d'une phase céramique bioactive sous forme de poudre. La phase céramique bioactive est un verre bioactif.

Dans une forme de réalisation de l'invention, le verre bioactif est constitué de 45% en poids de $SiO_2$, par rapport au poids total du verre bioactif, de 24,5% en poids de CaO, par rapport au poids total du verre bioactif, de 24,5% en poids de $Na_2O$, par rapport au poids total du verre bioactif et de 6% en poids de $P_2O_5$, par rapport au poids total du verre bioactif. Ce verre bioactif a la propriété de développer en sa surface, lorsqu'il est immergé dans un milieu physiologique, une couche d'hydroxyapatite carbonatée (HAC) de la famille des apatites. L'hydroxyapatite carbonatée a une structure proche de la partie minérale de l'os. Ce verre bioactif favorise particulièrement la formation osseuse. Ce verre bioactif comporte de plus des composants nécessaires à la repousse osseuse, en particulier des ions calcium et phosphore.

Un tel verre bioactif peut être obtenu par la voie classique décrite ci-après : des poudres de $SiO_2$, $CaCO_3$, $Na_2CO_3$ et $P_2O_5$ sont pesées et mélangées. Les mélanges sont ensuite placés dans des creusets de platine et portés à 950°C dans un four pour une première étape de synthèse, à savoir la décarbonatation, qui dure environ 5 heures. Suit une deuxième étape, à savoir la fusion des mélanges, qui a lieu à 1400°C pendant une durée d'environ 4 heures. On réalise ensuite une trempe dans l'eau du mélange obtenu. Le verre bioactif ainsi obtenu peut être broyé et tamisé. De préférence, on utilise selon la présente invention du verre bioactif dont la taille moyenne des particules va de 1 à 15 microns, de préférence de 3 à 4 microns. La masse volumique du verre bioactif va de préférence de 2,55 à 2,70 g/cm$^3$, de préférence encore de 2,65 à 2,68 g/cm$^3$.

Un verre bioactif convenant à la présente invention est disponible commercialement sous la dénomination « 4555® » auprès de la société USBiomaterials Corporation.

Selon le procédé de l'invention, la phase céramique bioactive est préparée sous forme de poudre. Pour cela, les matières premières constituant cette phase sont broyées le cas échéant, selon des techniques de broyage classiques, pour obtenir des particules. De préférence, la poudre de la phase céramique bioactive présente une granulométrie allant de 1 à 15 microns, de préférence allant de 3 à 4 microns.

Dans une deuxième étape du procédé selon l'invention, à savoir l'étape b°), la poudre de phase céramique bioactive est mise en suspension dans un solvant. Le solvant de l'étape b°) est l'acétone.

La mise en suspension de l'étape b°) peut être réalisée de façon classique, par simple mélange, par exemple en utilisant un mélangeur mécanique tel que l'agitateur à hélice du type «IKA® RW 20 » de chez IKA-WERKE GMBH & CO.KG ou encore sous agitation magnétique. De préférence, l'étape de mise en suspension est réalisée à température ambiante (environ 20°C).

Dans une troisième étape du procédé selon l'invention, à savoir l'étape c°), on ajoute à la suspension obtenue en b°) un polymère biorésorbable, et on mélange jusqu'à obtention d'une dispersion visqueuse homogène de ladite poudre de céramique dans une solution composée dudit solvant et dudit polymère.

Le polymère biorésorbable est un copolymère d'acide poly(L-lactique-co-D,L-lactique). De préférence, ledit copolymère d'acide poly(L-lactique-co-D,L-lactique) est composé de 70% d'acide poly(L,lactique) et de 30% d'un mélange racémique d'acide poly(D,-lactique) 50/50 : un copolymère d'une telle composition est disponible commercialement sous la dénomination « Resomer LR 706 ® » auprès de la société Bohringer.

Le polymère biorésorbable est ajouté à ladite suspension en une proportion allant de 1 à 90%, en poids, de préférence allant de 5 à 80%, en poids, par rapport au poids du mélange constitué de la phase céramique et du polymère biorésorbable.

Ainsi, de préférence, la proportion de phase céramique dans le matériau composite obtenu selon le procédé de l'invention peut aller jusqu'à 80%, en poids, par rapport au poids du matériau composite.

Dans un mode de réalisation de l'invention, le polymère biorésorbable est ajouté sous forme d'une poudre présentant une granulométrie allant de 800 à 2000 microns.

De préférence, l'étape c°) est effectuée sous agitation, par exemple sous agitation mécanique ou magnétique. De préférence, cette agitation doit permettre la solubilisation totale du polymère biorésorbable dans le solvant. Ainsi, de préférence, l'agitation est continuée jusqu'à solubilisation totale du polymère biorésorbable dans le solvant : par exemple, dans le cas où le polymère biorésorbable a été ajouté sous forme de poudre dans la suspension obtenue à l'étape b°), l'agitation est de préférence continuée jusqu'à solubilisation totale des particules de polymère biorésorbable dans le solvant. De préférence, l'agitation permet également l'homogénéisation du mélange complet, à savoir le solvant, la poudre de céramique et le polymère biorésorbable. Ainsi, de préférence, la solubilisation complète et l'homogénéisation permettent d'obtenir une dispersion visqueuse, c'est-à-dire des particules de phase céramique bioactive, par exemple de verre bioactif, en suspension dans une solution composée de solvant et de polymère biorésorbable.

Dans une forme préférée de réalisation de l'invention, l'agitation est continuée jusqu'à obtention d'une dispersion visqueuse présentant sensiblement la consistance d'un miel coulant à température ambiante (environ 20°C).

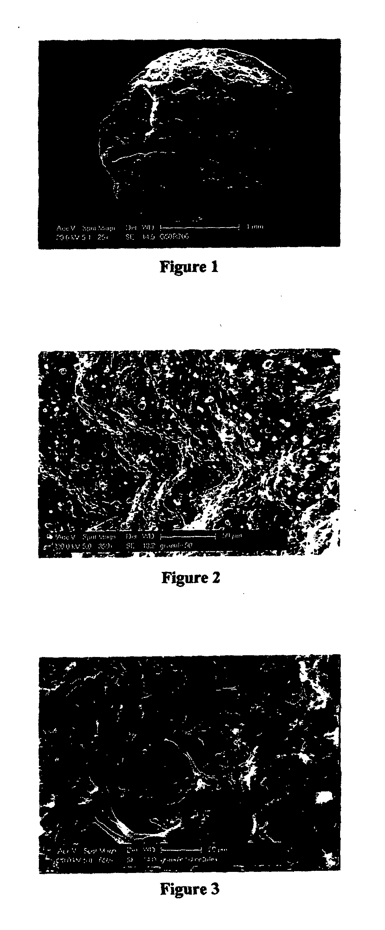

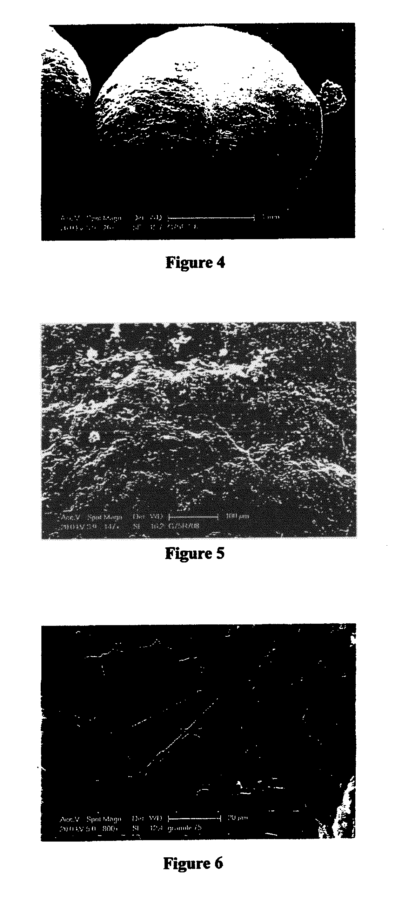

Une telle homogénéisation de la dispersion et une telle nature visqueuse de la dispersion, en particulier obtenues grâce à l'ordre spécifique des étapes a°)-c°), c'est-à-dire a°) puis b°) puis c°) du procédé selon l'invention, permettent d'obtenir au final un matériau composite très homogène, c'est-à-dire dans lequel les particules de céramique sont réparties uniformément et régulièrement au sein de la phase polymère, comme il ressortira clairement de la description des figures 2 et 5 ci-après.

Dans une quatrième étape du procédé selon l'invention, on réalise une précipitation de la dispersion obtenue en c°) dans une solution aqueuse pour obtenir un matériau composite homogène. Le solvant des étapes b°) et c°) est généralement éliminé lors de cette étape de précipitation, par exemple par évaporation.

Le matériau composite obtenu selon le procédé de l'invention comprend de préférence au moins 5% en poids, de préférence de 30% à 80%, en poids, d'une phase céramique, par rapport au poids total du matériau composite.

Dans une forme de réalisation du procédé de l'invention, la dispersion est précipitée sous forme d'amas dans de l'eau, par exemple par coulage de la dispersion obtenue en c°) dans des réservoirs d'eau. De préférence, l'amas de matériau composite obtenu est ensuite séché et broyé pour obtenir des granules. De préférence, on broie l'amas séché pour obtenir des granules présentant un diamètre moyen allant de 0,1 à 2 mm, de préférence encore de allant de 0,3 à 1 mm.

Dans une autre forme de réalisation du procédé de l'invention, ladite dispersion est précipitée sous forme de gouttelettes dans de l'eau, lesdites gouttelettes de matériau composite obtenues étant ensuite séchées pour obtenir des granules. Les granules directement obtenues par précipitation de gouttelettes présentent de préférence une forme sensiblement sphérique. Alternativement, les gouttelettes séchées peuvent être broyées pour obtenir des granules.

Les granules ainsi obtenus présentent de préférence un diamètre moyen allant de 0,1 à 5 mm, de préférence encore de allant de 1 à 2 mm.

L'étape d°) de précipitation par voie humide peut ainsi comprendre une étape de coulage de la dispersion obtenue en c°) dans une burette munie d'un robinet et mise en place d'un système de goutte à goutte au-dessus d'un réservoir d'eau.

Ainsi, les granules obtenus selon le procédé de l'invention, par précipitation d'un amas ou de gouttelettes, comprennent de préférence au moins 5% en poids, de préférence de 30% à 80%, en poids, d'une phase céramique, par rapport au poids total de la granule.

Le matériau composite et/ou les granules obtenus par le procédé selon l'invention présentent une composition homogène, c'est-à-dire que les particules de phase céramique, par exemple de verre bioactif, sont dispersées de façon très régulière au sein de la matrice en polymère biorésorbable.

Les figures 2 et 5 (voir également les exemples 1, 2 et 3) sont des vues prises au microscope électronique à balayage du matériau composite selon l'invention à différentes compositions de phase céramique et de phase polymérique, montrant la répartition des particules de céramique bioactive au sein de la matrice en polymère. Il apparaît que les particules de céramique bioactive sont réparties de façon homogène dans l'ensemble de la matrice. En particulier, grâce au procédé selon l'invention, la précipitation lors de l'étape d°) de la dispersion homogène obtenue en c°) dans l'eau permet aux particules de phase céramique d'être liées chimiquement à la matrice en polymère biorésorbable et non pas uniquement mécaniquement, comme dans les matériaux de l'art antérieur.

Il est ainsi possible de fabriquer des compositions pulvérulentes de granules de matériau composite homogène obtenues selon le procédé selon l'invention et d'utiliser le matériau composite soit directement, soit sous la forme de telles compositions pulvérulentes, dans des techniques de transformation nécessitant une étape de préchauffage, sans entraîner de séparation ou de ségrégation au sein du matériau composite ou des granules de matériau composite entre la phase céramique et la phase organique de polymère biorésorbable lors de cette étape de préchauffage.

Le matériau composite en particulier sous la forme de granules, peut être utilisé efficacement dans des procédés de mise en forme par des techniques nécessitant au moins une étape de chauffage dudit matériau pour fabriquer des dispositifs médicaux implantables.

Ainsi, les dispositifs médicaux implantables peuvent être fabriqués selon le procédé de fabrication suivant :

- 1°) on dispose d'un matériau composite tel que décrit ci-dessus,
- 2°) on chauffe ledit matériau composite pour obtenir une pâte homogène,
- 3°) on coule ladite pâte homogène dans un moule,
- 4°) après refroidissement, le dispositif médical implantable est obtenu par démoulage.

Lors de l'étape 2°) de chauffage du matériau composite la température peut aller de 130 à 170°C. Du fait de la nature particulièrement homogène de la composition du matériau composite le matériau composite, sous l'action de la chaleur se transforme en une pâte qui reste elle-même homogène. Il ne se produit pas de séparation ou de ségrégation des phases, céramique d'une part, et polymère d'autre part.

Par exemple, l'étape 3°) peut être réalisée par une technique de transformation choisie parmi le moulage par injection, le moulage par injection transfert, le moulage par compression, le moulage par extrusion. Lors de cette étape, la pâte obtenue à l'étape 2°), du fait qu'elle est particulièrement homogène, est tout à fait adaptée à être traitée par la machine de la technique considérée, par exemple la filière de l'extrudeuse.

On obtient ainsi un dispositif médical implantable présentant des propriétés biologiques et mécaniques remarquables. Ce dispositif médical implantable peut par exemple se présenter sous la forme d'une vis d'interférence, d'une pine, de cages intervertébrales cervicales et lombaires, de plaques cervicales, d'ancres ou d'agrafes.

Le procédé de préparation du matériau composite selon l'invention et le matériau composite permettent de fabriquer des dispositifs médicaux implantables particulièrement résistants, biorésorbables et favorisant la formation osseuse.

Le matériau composite, en particulier sous la forme de granules, peut être mis en forme par moulage par injection, moulage par injection transfert, moulage par compression, par extrusion ou par usinage microtechnique pour fabriquer des dispositifs médicaux implantables biorésorbables de formes complexes, destinés à être implantés dans le corps humain ou animal, comme par exemple des vis d'interférence, des pines, des cages intervertébrales cervicales et lombaires, des plaques cervicales, des ancres, des agrafes, etc....

Des exemples illustrant la présente invention vont maintenant être donnés, dans lesquels :

- la figure 1 est une micrographie d'une granule de matériau composite obtenue selon le procédé de l'invention, comprenant 50% en poids de phase céramique et 50% en poids de phase polymère, par rapport au poids du granule, réalisée par microscopie électronique à balayage (840 A LGS de chez JEOL) avec un agrandissement de 25.
- la figure 2 est une micrographie de la surface du granule de la figure 1 obtenue par microscopie électronique à balayage (840 A LGS de chez JEOL) avec un agrandissement de 350.
- la figure 3 est une vue obtenue par microscopie électronique à balayage (840 A LGS de chez JEOL) avec un agrandissement de 650, du granule de la figure 1 recouvert de cellules (ostéoblastes MG-63),
- la figure 4 est une micrographie d'un granule de matériau composite obtenue selon le procédé de l'invention, comprenant 75% en poids de phase céramique et 25% en poids de phase polymère, par rapport au poids du granule, obtenue par microscopie électronique à balayage (840 A LGS de chez JEOL) avec un agrandissement de 26.
- la figure 5 est une micrographie de la surface du granule de la figure 4 obtenue par microscopie électronique à balayage (840 A LGS de chez JEOL) avec un agrandissement de 147,
- la figure 6 est une vue obtenue par microscopie électronique à balayage (840 A LGS de chez JEOL) avec un agrandissement de 800, du granule de la figure 4 recouverte de cellules (ostéoblastes MG-63).

EXEMPLE 1 :

Préparation d'un matériau composite de composition homogène selon l'invention comprenant 50% en poids de phase céramique et 50% en poids de phase polymère par rapport au poids du matériau composite :

On dispose pour la phase céramique bioactive d'une poudre de verre bioactif composée de 45% de $SiO_2$, 24.5 % de CaO et $Na_2O$ et 6% de $P_2O_5$ en pourcentages massiques.

Ce verre bioactif est obtenu selon la méthode de préparation suivante: des poudres de $SiO_2$, $CaCO_3$ $Na_2CO_3$ et $P_2O_5$ sont pesées et mélangées. Les mélanges sont ensuite placés dans des creusets de platine et portés à 950°C dans un four pour une première étape de synthèse, à savoir la décarbonatation, qui dure environ 5 heures. Suit une deuxième étape, à savoir la fusion des mélanges, qui a lieu à 1400°C pendant une durée d'environ 4 heures. On réalise ensuite une trempe dans l'eau du mélange obtenu.

Le verre bioactif ainsi obtenu est broyé et tamisé pour obtenir des particules dont la taille moyenne va de 3 à 4 microns. La masse volumique de ce verre bioactif va de 2,65 à 2,68 $g/cm^3$.

100 g de particules de verre bioactif ainsi obtenues sont mises en suspension dans l'acétone.

On dispose d'un copolymère d'acide poly(L-lactique-co-D,L-lactique) composé de 70% d'acide poly(L,lactique) et de 30% d'un mélange racémique d'acide poly(D,-lactique) 50/50 : un copolymère d'une telle composition est disponible commercialement sous forme de poudre de granulométrie allant de 800 à 2000 microns sous la dénomination « Resomer LR 706 ®» auprès de la société Bohringer. 100 g de ce copolymère d'acide poly(L-lactique-co-D,L-lactique) sont mis en solution dans la suspension du verre bioactif et mélangé à l'aide d'un agitateur ou d'un mélangeur pendant 5 heures jusqu'à solubilisation totale du copolymère dans l'acétone. Après 5 h, une dispersion visqueuse, ayant la consistance de miel coulant à température ambiante (environ 20°C), et très homogène est obtenue.

La dispersion visqueuse est ensuite coulée dans une burette munie d'un robinet. Le débit de la dispersion est réglé par le robinet de sorte d'obtenir un goutte à goutte. Les gouttelettes sont précipitées dans de l'eau, étape au cours de laquelle l'acétone est éliminée par évaporation. Puis les granules obtenus par la précipitation des gouttelettes sont séchés à atmosphère ambiante (environ 20°C) pendant 2 h puis dans une étuve à 40°C pendant 24h. On obtient ainsi des granules de forme sensiblement sphérique : un tel granule est visible sur la figure 1 qui est une micrographie d'un tel granule obtenu avec un microscope électronique à balayage de type 840 A LGS de chez JEOL avec un agrandissement de 25.

Ces granules de matériau composite sont de composition homogène : la composition homogène de ces granules est visible sur la figure 2 qui est une micrographie de la surface d'un tel granule obtenu avec un microscope électronique à balayage de type 840 A LGS de chez JEOL avec un agrandissement 350. Sur cette micrographie, les particules de verre bioactif apparaissent clairement sous la forme de petites tâches blanches, dispersées de façon régulière au sein de la matrice en polymère. Chaque granule présente une composition de 50% en poids de verre bioactif sous la forme de particules et 50% en poids de polymère biorésorbable sous la forme d'une matrice dans laquelle lesdites particules de verre bioactif sont réparties de façon régulière.

Ces granules présentent une granulométrie, ou encore une taille moyenne des granules, allant de 1 à 2 mm.

Il a été vérifié par des mesures de masses volumiques absolues par pycnomètre à Hélium (Micromeritics accu Pyc 1330) que les granules ainsi obtenus sont homogènes comme le montre tableau 1 ci-dessous.

Tableau 1 : Evolution de la masse volumique absolue et de la masse volumique calculée en fonction du pourcentage massique en verre bioactif dans le granule.

| Pourcentage **massique** « Resomer LR 706 ® »/verre bioactif | 100/0 | 80/20 | 50/50 | 40/60 | 25/75 | 0/100 |
|---|---|---|---|---|---|---|
| Masse volumique absolue (mesurée) (g/cm$^3$) | 1,27 | 1,47 | 1,76 | 1,89 | 2,17 | 2,67 |
| Masse volumique calculée (théorique) (g/cm$^3$) | 1,27 | 1,42 | 1,72 | 1,85 | 2,09 | 2,67 |

Dans le matériau composite obtenu, la composition de verre bioactif mesurée, c'est-à-dire la proportion de verre bioactif dans le matériau composite final, varie très peu, c'est-à-dire est très peu éloignée, de la composition de verre bioactif attendue compte tenu de la masse volumique calculée à partir des proportions de verre bioactif et de polymère biorésorbable de départ. Une variation de 0 à 3% a été constatée dans le cas des mélanges de polymère « Resomer LR 706 ® »/verre bioactif de 80/20 ; 50/50 ; 40/60 ; 25/75 en pourcentage massique.

Ces résultats ont été confirmés par calcul à partir de la loi des mélanges :

$$\frac{1}{\rho_{eq}} = \frac{x_b}{\rho_b} + \frac{x_p}{\rho_p}$$

dans laquelle :

$\rho_{eq}$ : masse volumique absolue du mélange (g/cm$^3$)

$\rho_b$ : masse volumique du verre bioactif = 2,67 g/cm$^3$

$\rho_p$ : masse volumique du polymère « Resomer LR 706 ® » = 1,27 g/cm$^3$

$x_b$ : pourcentage massique du verre bioactif (%)

$x_p$ : pourcentage massique du polymère « Resomer LR 706 ® » (%)

Il a été vérifié que les granules ainsi obtenus présentent un carcatère bioactif. Ainsi, ces granules ont été immergés dans une solution SBF (Simulated Body Fluid) qui contient les mêmes ions aux mêmes concentrations que le plasma humain, de pH variant de 7,2 à 7,4.

Des analyses de diffraction des rayons X et de microscopie électronique à balayage montrent, après immersion des granules dans le SBF, la formation d'une phase d'hydroxyapatite cristallisée à la surface des granules. La formation de cette phase est caractéristique de la bioactivité des granules.

Par ailleurs, des observations au microscope à balayage électronique ont permis de vérifier que les cellules (ostéoblastes MG-63) adhèrent à la surface des granules : l'adhésion des cellules est visible sur la figure 3 qui est une vue obtenue avec un microscope électronique à balayage de type 840 A LGS de chez JEOL avec un agrandissement 650, d'un granule du présent exemple 1 recouvert de cellules. Ces cellules forment des prolongements cytoplasmiques au niveau des dénivellations des granules.

Ainsi, les granules obtenus selon cet exemple 1 sont particulièrement adaptés à la fabrication de dispositifs médicaux implantables de formes complexes, par des techniques de transformation nécessitant une étape préalable de chauffage. Les dispositifs médicaux implantables obtenus avec les granules de l'exemple 1 comprennent 50% en poids de phase céramique. Ils présentent ainsi des propriétés biologiques et mécaniques remarquables particulièrement avantageuses pour leur utilisation en tant qu'éléments de fixation de substituts osseux par exemple.

EXEMPLE 2 :

Préparation d'un matériau composite de composition homogène selon l'invention comprenant 75% en poids de phase céramique et 25% en poids de phase polymère par rapport au poids du matériau composite :

Une dispersion visqueuse a été préparée selon la méthode de l'exemple 1 en utilisant le même verre bioactif que dans l'exemple 1 et le même polymère biorésorbable que dans l'exemple 1 mais en utilisant respectivement 75 g de verre bioactif et 25 g de polymère biorésorbable.

La dispersion visqueuse est ensuite coulée directement dans un réservoir d'eau afin d'obtenir un amas de précipité composé du verre bioactif et du polymère biorésorbable. L'amas de matériau composite obtenu après cette précipitation présente une composition homogène, visible sur la figure 5 qui est une micrographie de la surface d'un tel amas avec un microscope électronique à balayage de type 840 A LGS de chez JEOL avec un agrandissement 147. Sur cette micrographie, les particules de verre bioactif apparaissent clairement sous la forme de petites tâches blanches, dispersées de façon régulière au sein de la matrice en polymère. Cet amas présente une composition de 75% en poids de verre bioactif sous la forme de particules et 25% en poids de polymère biorésorbable sous la forme d'une matrice dans laquelle lesdites particules de verre bioactif sont réparties de façon régulière.

Cet amas est ensuite séché puis broyé. On obtient ainsi des granules de matériau composite comprenant du verre bioactif et du polymère biorésorbable, présentant une granulométrie, c'est-à-dire une taille moyenne des granules, allant de 300 à 2000 microns. On obtient ainsi des granules de forme sensiblement sphérique : un tel granule est visible sur la figure 4 qui est une micrographie d'un tel granule obtenu avec un microscope électronique à balayage de type 840 A LGS de chez JEOL avec un agrandissement 26.

La bioactivité de ces granules a été vérifiée et confirmée de la même façon que dans l'exemple 1. En particulier, des observations au microscope à balayage électronique ont permis de vérifier que les cellules adhèrent à la surface des granules : l'adhésion des cellules est visible sur la figure 6 qui est une vue obtenue avec un microscope électronique à balayage de type 840 A LGS de chez JEOL avec un agrandissement de 800, d'un granule du présent exemple 2 recouvert de cellules. Ces cellules forment des prolongements cytoplasmiques au niveau des dénivellations des granules.

EXEMPLE 3

Fabrication d'un dispositif médical implantable à partir des granules obtenus à l'exemple 1 :

Une composition pulvérulente de granules de matériau composite obtenus à l'exemple 1 est versée dans un bol de transfert. La composition y est malaxée et chauffée. Grâce à la nature particulièrement homogène de la composition des granules de l'exemple 1, le traitement mécanique et thermique fournit une pâte fondante qui reste homogène. Cette pâte homogène sans bulle est transférée dans un moule vers un orifice. La pâte est poussée par pression à travers un orifice par un piston et vient remplir un moule fermé et refroidi. Au contact des parois froides, la pâte prend la forme du moule et se solidifie. Le moule s'ouvre ensuite pour faire sortir la pièce.

On obtient après démoulage des produits finis ou semi-finis de formes complexes en une seule opération.

De la même façon, une composition pulvérulente de granules de matériau composite de composition homogène selon l'invention comprenant 20% en poids de phase céramique et 80% en poids de phase polymère par rapport au poids du matériau composite a été utilisée pour fabriquer un dispositif médical implantable par moulage par injection transfert ou moulage par injection.

Par exemple des plaques cervicales, des cages intervertébrales cervicales et lombaires, des vis de fixation et des vis d'interférence sont fabriquées par moulage par injection transfert ou moulage par injection selon les conditions opératoires suivantes :

Paramètres de transformation :

    Pression : 90 - 110 bars
    Température : 135 - 165°C

Propriétés mécaniques des produits obtenus :

Des essais de compression sur une machine INSTRON ont été réalisés sur des éprouvettes de dimension 10mmX10mmX4mm (selon la norme ISO 604) de matériaux composites obtenus selon l'exemple 1 avec une vitesse de la traverse de 0,5 mm/min. Les résultats obtenus sont présentés dans le tableau 2 ci-dessous :

Tableau 2 : Caractéristiques en compression pour les matériaux composites de composition 20% en masse de verre bioactif / 80% en « Resomer LR706® », 50% en masse de verre bioactif / 50% en masse de « Resomer LR706® », et le polymère « Resomer LR706® » seul.

| Matériau testé | « Résomer LR 706® » | 20/80 (verre bioacti/« Resomer LR706® ») | 50/50 (verre bioacti/« Resomer LR706® ») | Os cortical |
|---|---|---|---|---|
| Module de Young (GPa) | 4 | 6 | 10 | **20 \*** |
| Limite D'élasticité (MPa) | 80 | 87 | 84 | - |
| Contrainte à la rupture en compression (MPa) | 84 | 140 | 149 | **150\*** |
| * Valeur de référence citée dans la littérature | | | | |

Les mesures du module d'Young ont été réalisées par la méthode de résonance avec un appareil de type Grindo Sonic. Cette méthode utilise le principe de l'excitation par choc. L'énergie acquise par une pièce sollicitée par un choc se dissipe sous forme de vibrations qui seront, entre autre, fonction des caractéristiques du matériau. La mesure de la fréquence propre de résonance d'éprouvettes de géométrie simple permet de déterminer le module.

Les valeurs obtenues pour le module d'Young et les essais en compression montrent que le matériau composite selon l'invention possède des propriétés mécaniques plus importantes que le polymère seul. Plus la concentration en bioverre dans le composite augmente, plus le module d'Young augmente. Le matériau composite possède des propriétés mécaniques proches de celles de l'os autant en élasticité (module de Young) qu'en résistance en compression. Le matériau composite possède une résistance mécanique de l'ordre de 149 MPa très proche de celle de l'os cortical (150 MPa).

**Revendications**

1.  Procédé de préparation d'un matériau composite de composition homogène, comprenant au moins une phase céramique bioactive et au moins un polymère biorésorbable, **caractérisé en ce qu'**il comprend les étapes suivantes :

    a°) on dispose d'une phase céramique bioactive sous forme de poudre, ladite phase céramique bioactive étant un verre bioactif,
    b°) on met ladite poudre de céramique bioactive en suspension dans un solvant, le solvant étant l'acétone,
    c°) on ajoute à la suspension obtenue en b°) un polymère biorésorbable, ledit polymère biorésorbable étant un copolymère d'acide poly(L-lactique-co-D,L-lactique), ledit polymère biorésorbable est ajouté à l'étape c°) dans une proportion allant de 1 à 90 % en poids par rapport au poids du mélange constitué de la phase céramique et du polymère biorésorbable, et on mélange jusqu'à obtention d'une dispersion visqueuse homogène de ladite poudre de céramique bioactive dans une solution composée dudit solvant et dudit polymère,
    d°) on réalise une précipitation de la dispersion obtenue en c°) dans une solution aqueuse pour obtenir un matériau composite homogène.

2.  Procédé selon la revendication 1, **caractérisé en ce que**, lors de l'étape d°) ladite dispersion est précipitée sous forme d'amas dans de l'eau, l'amas de matériau composite obtenu étant ensuite séché et broyé pour obtenir des granules.

3.  Procédé selon la revendication 1, **caractérisé en ce que**, lors de l'étape d°), ladite dispersion est précipitée sous forme de gouttelettes dans de l'eau, lesdites gouttelettes de matériau composite obtenues étant ensuite séchées pour obtenir des granules.

4.  Procédé selon la revendication 3, **caractérisé en ce que** les gouttelettes séchées sont broyées pour obtenir des granules.

5.  Procédé selon l'une des revendications précédentes, **caractérisé en ce que** ledit verre bioactif est constitué de 45% en poids de $SiO_2$, par rapport au poids total du verre bioactif, de 24,5% en poids de CaO, par rapport au poids total du verre bioactif, de 24,5% en poids de $Na_2O$, par rapport au poids total du verre bioactif et de 6% en poids de $P_2O_5$, par rapport au poids total du verre bioactif.

**6.** Procédé selon l'une quelconque des revendications précédentes, **caractérisé en ce que** la poudre de la phase céramique bioactive de l'étape a°) présente une granulométrie allant de 1 à 15, de préférence allant de 3 à 4 microns.

**7.** Procédé selon l'une des revendications précédentes, **caractérisé en ce que** ledit copolymère d'acide poly(L-lactique-co-D,L-lactique) est composé de 70% d'acide poly(L,lactique) et de 30% d'un mélange racémique d'acide poly(D,-lactique) 50/50.

**8.** Procédé selon l'une quelconque des revendications précédentes, **caractérisé en ce que** ledit polymère biorésorbable est ajouté à l'étape c°) dans une proportion allant de 5 à 80%, en poids, par rapport au poids du mélange constitué de la phase céramique et du polymère biorésorbable.

**9.** Procédé selon l'une quelconque des revendications précédentes, **caractérisé en ce que** l'étape c°) est effectuée sous agitation.

**10.** Procédé selon la revendication précédente, **caractérisé en ce que** l'agitation est continuée jusqu'à solubilisation totale du polymère biorésorbable dans le solvant.

**11.** Procédé selon l'une quelconque des revendications 3 à 10, **caractérisé en ce que** l'étape d°) comprend une étape de coulage de la dispersion obtenue en c°) dans une burette munie d'un robinet et mise en place d'un système de goutte à goutte au-dessus d'un réservoir d'eau.

**Patentansprüche**

**1.** Verfahren zur Herstellung eines Verbundmaterials mit homogener Zusammensetzung, umfassend mindestens eine biologisch aktive keramische Phase und mindestens ein biologisch resorbierbares Polymer, **dadurch gekennzeichnet, dass** es die folgenden Schritte umfasst:

a°) Anordnen einer biologisch aktiven keramischen Phase in Form eines Pulvers, wobei die biologisch aktive keramische Phase ein biologisch aktives Glas ist,
b°) Suspendieren des Pulvers der biologisch aktiven keramischen Phase in einem Lösemittel, wobei das Lösemittel Aceton ist,
c°) Zugeben zur Suspension, die in b°) erhalten wurde, eines biologisch resorbierbaren Polymers, wobei das biologisch resorbierbare Polymer ein Copolymer von Poly(L-lactid-co-D,L-lactid)-Säure ist, wobei das biologisch resorbierbare Polymer in Schritt c°) in einer Proportion zugegeben wird, die von 1 bis 90 % mit Bezug auf das Gewicht der Mischung reicht, die aus der keramischen Phase und dem biologisch resorbierbaren Polymer besteht, und Mischen, bis eine homogene visköse Dispersion des Pulvers aus biologisch aktiver Keramik in einer Lösung erhalten wird, die aus dem Lösemittel und dem Polymer zusammengesetzt ist,
d°) Durchführen einer Ausfällung der Dispersion, die in c°) erhalten wurde, in einer wässerigen Lösung, um ein homogenes Verbundmaterial zu erhalten.

**2.** Verfahren nach Anspruch 1, **dadurch gekennzeichnet, dass** in Schritt d°) die Dispersion in Form eines Clusters in Wasser ausgefällt wird, wobei der erhaltene Cluster aus Verbundmaterial dann getrocknet und gemahlen wird, um Körner zu erhalten.

**3.** Verfahren nach Anspruch 1, **dadurch gekennzeichnet, dass** in Schritt d°) die Dispersion in Form von Tröpfchen in Wasser ausgefällt wird, wobei die erhaltenen Tröpfchen aus Verbundmaterial dann getrocknet und gemahlen werden, um Körner zu erhalten.

**4.** Verfahren nach Anspruch 3, **dadurch gekennzeichnet, dass** die getrockneten Tröpfchen gemahlen werden, um Körner zu erhalten.

**5.** Verfahren nach einem der vorhergehenden Ansprüche, **dadurch gekennzeichnet, dass** das biologisch aktive Glas aus 45 Gew% $SiO_2$ mit Bezug auf das Gesamtgewicht des biologisch aktiven Glases, aus 24,5 Gew% CaO mit Bezug auf das Gesamtgewicht des biologisch aktiven Glases, aus 24,5 Gew% Na$_2$O mit Bezug auf das Gesamtgewicht des biologisch aktiven Glases und aus 6 Gew% P$_2$O$_5$ mit Bezug auf das Gesamtgewicht des biologisch aktiven Glases besteht.

6. Verfahren nach einem der vorhergehenden Ansprüche, **dadurch gekennzeichnet, dass** das Pulver der biologisch aktiven keramischen Phase von Schritt a°) eine Granulometrie aufweist, die von 1 bis 15, vorzugsweise von 3 bis 4 Mikrometer reicht.

7. Verfahren nach einem der vorhergehenden Ansprüche, **dadurch gekennzeichnet, dass** das Copolymer von Poly(L-lactid-co-D,L-lactid)-Säure aus 70 % Poly(L,lactid) Säure und aus 30 % einer racemischen Mischung aus Poly(D,-lactid) Säure 50/50 zusammengesetzt ist.

8. Verfahren nach einem der vorhergehenden Ansprüche, **dadurch gekennzeichnet, dass** das biologisch resorbierbare Polymer in Schritt c°) in einer Proportion zugegeben wird, die von 5 bis 80 Gew% mit Bezug auf die Mischung reicht, die aus der keramischen Phase und dem biologisch resorbierbaren Polymer besteht.

9. Verfahren nach einem der vorhergehenden Ansprüche, **dadurch gekennzeichnet, dass** der Schritt c°) unter Rühren durchgeführt wird.

10. Verfahren nach dem vorhergehenden Anspruch, **dadurch gekennzeichnet, dass** das Rühren bis zur gesamten Solubilisierung des biologisch resorbierbaren Polymers im Lösemittel fortgesetzt wird.

11. Verfahren nach einem der Ansprüche 3 bis 10, **dadurch gekennzeichnet, dass** Schritt d°) einen Schritt des Giessens der Dispersion, die in c°) erhalten wurde, in eine Bürette, die mit einem Hahn ausgestattet ist, und des Anbringens eines Tropfsystems über einem Wasserbehälter umfasst.

**Claims**

1. A method for preparing a composite material having a uniform composition, comprising at least one bioactive ceramic phase and at least one bioresorbable polymer, **characterized in that** it comprises the following steps:

   a) a bioactive ceramic phase in powder form is obtained, said bioactive ceramic phase being a bioactive glass,
   b) said bioactive ceramic powder is suspended in a solvent, said solvent being acetone,
   c) a bioresorbable polymer is added to the suspension obtained in b) and mixed to produce a viscous uniform dispersion of said bioactive ceramic powder in a solution formed by said solvent and said polymer, said bioresorbable polymer being a copolymer of poly(L-lactic-co-D,L-lactic) acid and being added in step c) in a proportion of 1 to 90% by weight, of the weight of the mixture consisting of the ceramic phase and the bioresorbable polymer,
   d) the dispersion obtained in c) is precipitated in an aqueous solution in order to obtain a uniform composite material.

2. The method as claimed in claim 1, **characterized in that**, during step d), said dispersion is precipitated in the form of a cluster in water, the cluster of composite material obtained then being dried and ground to obtain granules.

3. The method as claimed in claim 1, **characterized in that**, during step d), said dispersion is precipitated in the form of droplets in water, said droplets of composite material obtained then being dried to obtain granules.

4. The method as claimed in claim 3, **characterized in that** the dried droplets are ground to obtain granules.

5. The method as claimed in any of the preceding claims, **characterized in that** said bioactive glass consists of 45% by weight of SiO$_2$, of the total weight of the bioactive glass, 24.5% by weight of CaO, of the total weight of the bioactive glass, 24.5% by weight of Na$_2$O, of the total weight of the bioactive glass and 6% by weight of P$_2$O$_5$, of the total weight of the bioactive glass.

6. The method as claimed in any one of the preceding claims, **characterized in that** the powder of the bioactive ceramic phase of step a) has a particle size distribution from 1 to 15, preferably from 3 to 4 microns.

7. The method as claimed in any of the preceding claims, **characterized in that** said copolymer of poly(L-lactic-co-D,L-lactic) acid comprises 70% of poly(L,lactic) acid and 30% of a 50/50 racemic mixture of poly(D,-lactic) acid.

8. The method as claimed in any of the preceding claims, **characterized in that** said bioresorbable polymer is added in step c) in a proportion of 5 to 80% by weight, of the weight of the mixture consisting of the ceramic phase and the bioresorbable polymer.

9. The method as claimed in any one of the preceding claims, **characterized in that** step c) is carried out with stirring.

10. The method as claimed in the preceding claim, **characterized in that** the stirring is continued until the total solubilization of the bioresorbable polymer in the solvent.

11. The method as claimed in any one of claims 3 to 10, **characterized in that** step d) comprises a step of pouring of the dispersion obtained in c) into a burette provided with a cock and installation of a drip system above a water tank.

**Figure 1**

**Figure 2**

**Figure 3**

**Figure 4**

**Figure 5**

**Figure 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5977204 A
- US 4192021 A
- WO 0132072 A
- US 2002115742 A

**Littérature non-brevet citée dans la description**

- **ZHANG K et al.** Processing and properties of porous poly(l-lactide)/bioactive glass composites. *BIOMATERIALS,* 01 Juin 2004, vol. 25 (13), 2489-2500
- **ITOH S et al.** Development of an artificial vertebral body using a novel biomaterial, hydroxyapatite/collagen composite. *BIOMATERIALS,* 01 Octobre 2002, vol. 23 (19), 3919-3926